# FASCICULE DE BREVET EUROPEEN

(11) **EP 1 104 400 B1**
(45) Date de publication et mention de la délivrance du brevet: **01.10.2003**
(21) Numéro de dépôt: 00900626.3
(22) Date de dépôt: 20.01.2000
(51) Int. Cl.: C07C 213/08, C07C 219/08

(54) **PROCEDE DE FABRICATION DE SOLUTIONS AQUEUSES DE SELS INSATURES D'AMMONIUM QUATERNAIRE**
VERFAHREN ZUR HERSTELLUNG WÄSSERIGER LÖSUNGEN VON UNGESÄTTIGTEN QUARTÄREN AMMONIUMSALZEN
METHOD FOR MAKING AQUEOUS SOLUTIONS OF UNSATURATED QUATERNARY AMMONIUM SALTS

(30) Priorité: 21.01.1999 FR 9900643
(43) Date de publication de la demande: 06.06.2001
(73) Titulaire: Atofina, 92800 Puteaux (FR)
(72) Inventeur: RIONDEL, Alain, F-57600 Forbach (FR); HERBST, Gilles, F-57350 Spicheren (FR); GROSIUS, Paul, F-57540 Petite-Rosselle (FR)
(74) Mandataire: Rieux, Michel
(86) Numéro de dépôt international: FR0000124
(87) Numéro de publication internationale: WO00043348

(56) Documents cités:
- EP-A- 0 250 325
- EP-A- 0 329 512
- EP-A- 0 428 970
- EP-A- 0 818 437
- WO-A-89/07588
- DE-A- 4 037 284
- US-A- 5 545 757

## Description

La présente invention porte sur la fabrication de solutions aqueuses de sels insaturés d'ammonium quaternaire (ci-après dénommés sels quaternaires), répondant à la formule (I) suivante : 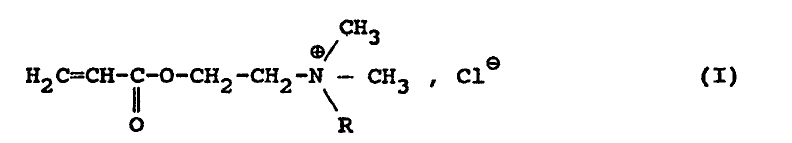 dans laquelle R représente méthyle ou benzyle,
par réaction, en présence d'eau, de l'acrylate de N,N-diméthylaminoéthyle (ADAME) avec un agent quaternisant de formule (II) :

R - Cl (II)

dans laquelle R est tel que défini ci-dessus.

On utilise des solutions aqueuses de sels quaternaires (I) pour préparer des polymères destinés à servir de floculants cationiques dans le traitement des eaux.

Le brevet européen EP-B-250 325 décrit un procédé de préparation de solutions aqueuse de sels quaternaires dont ceux de formule (I), procédé selon lequel, en présence d'au moins un inhibiteur de polymérisation :
- dans une première étape (a), on fait réagir l'ADAME avec 5 à 20% en poids de la quantité pondérale nécessaire à la réaction de l'agent quaternisant, ou, suivant une variante (a'), avec 5 à 20% en poids, par rapport au poids de l'ADAME, d'une solution aqueuse de sels quaternaires, laquelle comprend de 50 à 85% en poids de sels quaternaires ; et
- dans une deuxième étape (b), on ajoute en continu l'eau et l'agent quaternisant jusqu'à l'obtention de la concentration souhaitée de sels quaternaires dans l'eau.

Pendant les étapes (a) et (b), on maintient la température à une valeur comprise entre 30 et 60°C. De plus, pendant les étapes (a) et (b) et en particulier à l'approche de la fin de la réaction, on maintient dans le milieu réactionnel un courant de gaz oxygéné tel que le rapport en volume (ou débit volumétrique) de gaz total à la sortie du réacteur sur le volume (ou débit volumétrique) d'oxygène introduit à l'entrée de ce même réacteur est inférieur à 100.

Ce procédé permet de préparer des solutions aqueuses de sels quaternaires qui ont une stabilité à température ambiante supérieure à un an. Toutefois, on constate, dans ces solutions, une teneur particulièrement élevée d'impuretés, en particulier de 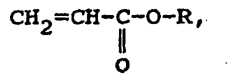 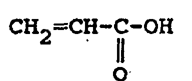 et d'ADAME. En outre, ce procédé nécessite des temps de réaction relativement longs, ce qui représente un inconvénient économique évident.

Dans la demande internationale WO 89/07 588, a alors été proposé un procédé destiné à réduire la formation des impuretés lors de la réaction de quaternisation. Conformément à ce procédé, la réaction est effectuée à une température comprise entre 10 et 80°C, et
- dans une première étape, on introduit dans le réacteur la totalité ou une partie de l'agent quaternisant nécessaire à la réaction, cet agent étant à l'état liquide dans les conditions de la réaction,
- ensuite, on ajoute l'ADAME, et
- dès que 0 à 30% de la stoechiométrie de l'ADAME ont été introduits dans le réacteur, on ajoute en continu et simultanément le reste d'agent quaternisant, le reste d'ADAME et l'eau jusqu'à l'obtention de la concentration souhaitée de sels quaternaires,
- et, dans le cas où l'agent quaternisant est introduit à l'état gazeux à la température de réaction, la réaction est effectuée en présence d'oxygène et on impose une pression de manière que l'agent quaternisant soit liquide à la température de réaction, et, en fin de réaction, on diminue progressivement la pression jusqu'à la pression atmosphérique et simultanément on impose un rapport en débit volumétrique de gaz total à la sortie du réacteur sur le débit volumétrique d'oxygène introduit dans le réacteur inférieur à, 100.

Le procédé ci-dessus selon WO 89/07 588 apporte des améliorations notables au procédé selon EP-B-250 325. Cependant, il est apparu que la pureté avec laquelle on obtient les sels quaternaires est encore insuffisante. Ainsi, au cours de la réaction de l'ADAME avec CH₃Cl en milieu aqueux, conduisant au sel désigné également dans ce qui suit par l'abréviation ADAMQUAT MC, il se forme, comme impuretés, outre l'acide acrylique (AA) formé par hydrolyse de l'ADAME, le dimère de l'ADAMQUAT MC, représenté par la formule (1) : 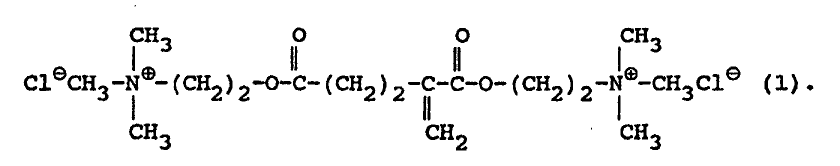 Grâce à une série de tests de réactivité en polymérisation, il a pu être démontré que ces impuretés affectaient la qualité des polymères cationiques dérivés de l'ADAMQUAT.

La Société déposante a donc recherché des conditions opératoires de préparation de solutions aqueuses du sel de formule (I), qui soient capables de minimiser des impuretés précitées, de façon à proposer un sel (I) en solution aqueuse de très haute qualité analytique.

Ce nouveau procédé, qui fait donc l'objet de la présente invention et qui présente l'avantage complémentaire important de fournir un produit de qualité constante, est caractérisé par le fait que l'on conduit ladite réaction en continu dans un réacteur tubulaire, avec introduction de l'agent quaternisant en pied de réacteur et introduction de l'ADAME et de l'eau en tête de réacteur, ladite réaction étant conduite à une température de 35 à 60°C, de préférence à une température de 40 à 50°C, et sous une pression de 10 à 20 bars, de préférence de 12 à 16 bars.

Par ailleurs, on conduit généralement la réaction avec un rapport molaire de CH₃Cl à l'ADAME qui est compris entre 1 et 1,5, en particulier entre 1,02 et 1,15, et un temps de séjour de 2 à 6 heures, en particulier de 2,5 à 3,5 heures. Quant au rapport des débits d'introduction eau/agent quaternisant, il est généralement compris entre 0,3 et 1,2, en particulier entre 0,5 et 0,9.

Le procédé selon la présente invention est par ailleurs avantageusement conduit en présence d'au moins un stabilisant, choisi notamment parmi le 3,5-ditert.-butyl-4-hydroxytoluène, l'éther méthylique d'hydroquinone, l'hydroquinone, le catéchol, le tert.-butyl catéchol et les mélanges de ces stabilisants, la teneur en agent(s) stabilisant(s) étant notamment de 20 à 2000 ppm, de préférence de 100 à 1200 ppm par rapport à la solution aqueuse de sel quaternaire (I).

Le procédé selon l'invention est en outre avantageusement conduit en présence d'au moins un agent séquestrant pour métaux, choisi notamment parmi l'acide diéthylène triamine penta acétique, le sel pentasodique de l'acide diéthylène triamine penta acétique, l'acide N-hydroxyéthyl-éthylène diamine triacétique et le sel trisodique de l'acide N-hydroxyéthyl-éthylène diamine triacétique, la teneur en agent(s) séquestrant(s) étant notamment de 1 à 100 ppm, de préférence de 5 à 30 ppm, par rapport à la solution aqueuse de sel quaternaire (I).

D'une manière générale, les agents séquestrants sont ajoutés sous la forme d'une solution aqueuse, car ils sont généralement disponibles sous cette forme. Ainsi, le sel pentasodique de l'acide diéthylène triamine penta acétique commercialisé sous la dénomination VERSENEX 80 se présente sous la forme d'une solution aqueuse à 40% en poids environ.

Le procédé selon l'invention permet notamment de préparer des solutions aqueuses ayant une concentration en sels quaternaires (I) de 50 à 85% en poids, et contenant des quantités très faibles d'impuretés, comme illustré dans le Tableau 1 ci-après.

Les Exemples suivants illustrent la présente invention sans toutefois en limiter la portée. Dans ces exemples, les pourcentages sont en poids sauf indication. contraire.

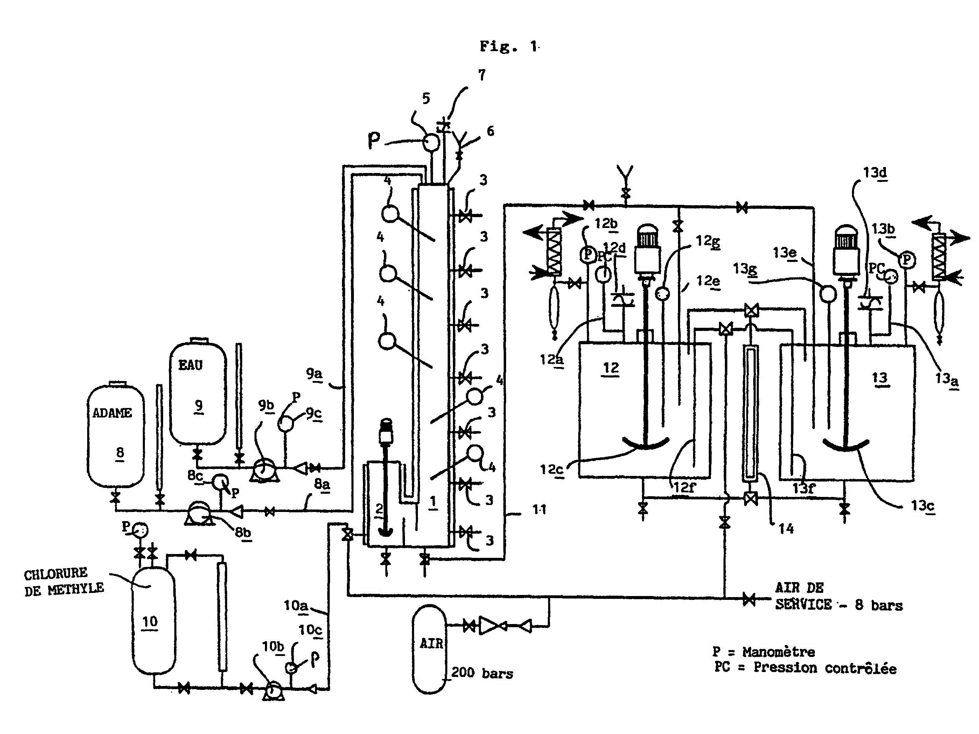

La Figure unique du dessin annexé représente l'installation utilisée pour la fabrication en continu des solutions aqueuses de chlorure d'acryloyloxytriméthyl-ammonium selon les Exemples. Cette installation est décrite à l'Exemple 1.

### EXEMPLE 1 : Fabrication en continu d'une solution aqueuse d'ADAMOUAT MC

On utilise un réacteur tubulaire (1) constitué d'un tube en acier inoxydable d'une capacité de 0,5 litre, comportant une zone (2) de prémélangeage des réactifs (1/10 du volume total) et équipé de sept prises d'échantillons (3), de cinq prises de température (4), d'un manomètre (5), d'une soupape d'équerre tarée à 17 bars (6), d'un disque d'éclatement taré à 25 bars (7), et de piquages permettant l'introduction des réactifs.

Chacun des trois bacs d'alimentation (8 ; 9 ; 10) respectivement en ADAME, eau et chlorure de méthyle est relié au réacteur tubulaire (1) par une conduite d'alimentation (respectivement 8a ; 9a ; 10a) avec interposition d'une pompe d'alimentation (8b ; 9b; 10b) et d'un manomètre (respectivement 8c ; 9c; 10c). La conduite (10a) d'alimentation en chlorure de méthyle débouche en pied de réacteur, et les deux conduites d'alimentation en ADAME et eau (respectivement (8a ; 9a) débouchent en tête de réacteur.

En fonctionnement, l'ADAMQUAT MC en solution aqueuse, dont la densité est supérieure à celle de l'ADAME et à celle du chlorure de méthyle, sort par le bas du réacteur (1) et est déversé par une conduite de sortie (11) dans l'une des deux capacités (12 ; 13) équipées chacune d'un régulateur de pression (12a ; 13a), d'un manomètre (12b ; 13b), d'un agitateur spécifique (12c ; 13c) fonctionnant sous pression, d'une soupape d'éclatement tarée à 25 bars (12d ; 13d), d'un dégazeur (12e, 13e), d'une introduction d'air (12f ; 13f), d'un niveau (14) et de prises de température (12g, 13g). La capacité dans laquelle est dirigé l'ADAMQUAT MC en solution aqueuse est utilisée comme dégazeur. Une fois remplie, elle est isolée et son contenu est dégazé puis déchargé, l'ADAMQUAT MC en solution aqueuse sortant du réacteur (1) étant alors dirigé sur l'autre capacité où il sera dégazé puis déchargé. Autrement dit, le réacteur fonctionne en continu et, pour l'opération de dégazage, le montage fonctionne en semi-continu.

Le mode opératoire est le suivant :

550 g d'ADAMQUAT MC 80 fini (solution aqueuse à 80%) sont chargés dans le réacteur (1). Le montage est mis sous pression (11 bars) à l'aide d'une bouteille d'air appauvri à 200 bars. Une fois le réacteur (1) mis à la température de travail souhaitée de 45°C, on démarre les trois pompes d'alimentation (8b ; 9b ; 10b) et on règle leur débit en fonction du temps de séjour moyen de 2,93 heures et du rapport molaire CH₃Cl/ADAME visé de 1,18. A cet effet, le débit d'introduction de l'ADAME est de 120 ml/h, celui de l'eau est de 35 ml/h, et celui du chlorure de méthyle, entre 49 et 53 ml/h. Dans ces conditions, le rapport molaire CH₃Cl/ADAME se situe entre 1,14 et 1,23 (rapport molaire moyen : 1,18), et le rapport des débits d'alimentation Eau/CH₃Cl, entre 0,71 et 0,78, et le temps de séjour est de 2,93 heures.

Les résultats sont donnés dans le Tableau 1 suivant. Le taux de conversion de l'ADAME est resté à 100% et le régime d'équilibre est atteint après 7,5 heures de fonctionnement.

Les techniques d'analyse étaient les suivantes :
- Le pourcentage d'ADAMQUAT MC a été déterminé par potentiométrie, permettant de doser les ions chlorure au moyen de nitrate d'argent.
- Les impuretés AA et dimère de l'ADAMQUAT MC ont été analysées par Chromatographie Liquide Haute Performance (HPLC).

### EXEMPLE 2

On a procédé comme à l'Exemple 1, excepté que l'on a diminué le rapport molaire CH₃Cl/ADAME. Les conditions opératoires et les résultats sont également rapportés dans le Tableau 1. Le taux de conversion de l'ADAME est resté à 100% pour une durée de mise en régime de 6 heures.

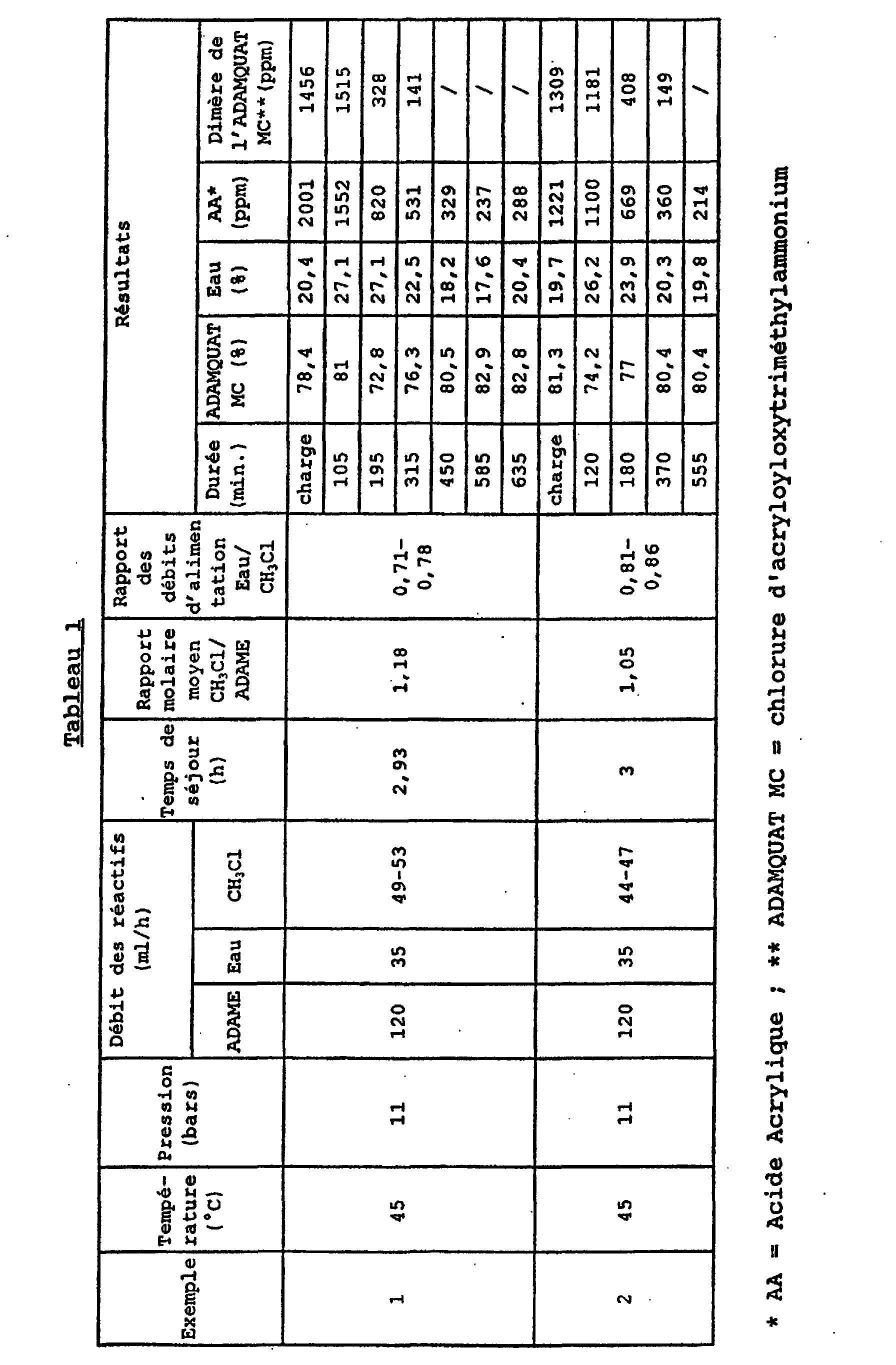

## Revendications

1. Procédé de fabrication d'une solution aqueuse du sel d'ammonium quaternaire insaturé, répondant à la formule (I) suivante 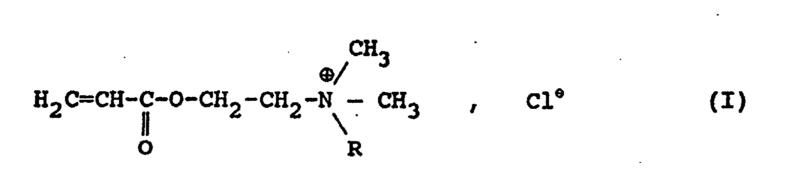 dans laquelle R représente un radical méthyle ou benzyle, par réaction, en présence d'eau, de l'acrylate de N,N-diméthylaminoéthyle (ADAME) avec un agent quaternisant de formule (II) :
R - Cl (II)
dans laquelle R est tel que défini ci-dessus,
**caractérisé par le fait que** l'on conduit ladite réaction en continu dans un réacteur tubulaire, avec introduction de l'agent quaternisant en pied de réacteur et introduction de l'ADAME et de l'eau en tête de réacteur, ladite réaction étant conduite à une température de 35 à 60°C et sous une pression de 10 à 20 bars.

2. Procédé selon la revendication 1, **caractérisé par le fait que** l'on conduit la réaction à une température de 40 à 50°C.

3. Procédé selon l'une des revendications 1 et 2, **caractérisé par le fait que** l'on conduit la réaction sous une pression de 12 à 16 bars.

4. Procédé selon l'une des revendications 1 à 3, **caractérisé par le fait que** l'on conduit la réaction avec un rapport molaire de CH₃Cl à l'ADAME qui est compris entre 1 et 1,5.

5. Procédé selon la revendication 4, **caractérisé par le fait que** l'on conduit la réaction avec un rapport molaire de l'agent quaternisant à l'ADAME qui est compris entre 1,02 et 1,15.

6. Procédé selon l'une des revendications 1 à 5, **caractérisé par le fait que** l'on conduit la réaction avec un rapport des débits d'introduction eau/agent quaternisant compris entre 0,3 et 1,2, en particulier entre 0,5 et 0,9.

7. Procédé selon l'une des revendications 1 à 6, **caractérisé par le fait que** l'on conduit la réaction avec un temps de séjour de 2 à 6 heures.

8. Procédé selon la revendication 7, **caractérisé par le fait que** l'on conduit la réaction avec un temps de séjour de 2,5 à 3,5 heures.

9. Procédé selon l'une des revendications 1 à 8, **caractérisé par le fait qu'**il est conduit en présence d'au moins un stabilisant, choisi notamment parmi le 3,5-ditert.-butyl-4-hydroxytoluène, l'éther méthylique d'hydroquinone, l'hydroquinone, le catéchol, le tert.-butyl catéchol et les mélanges de ces stabilisants, la teneur en agent(s) stabilisant(s) étant notamment de 20 à 200 ppm, de préférence de 100 à 1200 ppm par rapport à la solution aqueuse de sel quaternaire (I).

10. Procédé selon la revendication 9, **caractérisé par le fait qu'**il est conduit en outre en présence d'au moins un agent séquestrant pour métaux, choisi notamment parmi l'acide diéthylène triamine penta acétique, le sel pentasodique de l'acide diéthylène triamine penta acétique, l'acide N-hydroxyéthyl-éthylène diamine triacétique et le sel trisodique de l'acide N-hydroxyéthyl-éthylène diamine triacétique, la teneur en agent(s) séquestrant(s) étant notamment de 1 à 100 ppm, de préférence de 5 à 30 ppm, par rapport à la solution aqueuse de sel quaternaire (I).

## Patentansprüche

1. Verfahren zur Herstellung einer wässrigen Lösung eines ungesättigten quartären Ammoniumsalzes der folgenden Formel (I): 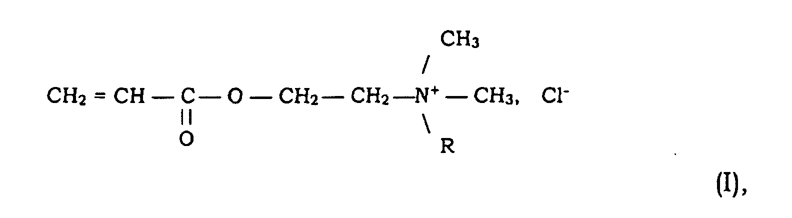 worin R Methyl oder Benzyl bedeutet, durch Umsetzung von N,N-Dimethylaminoethylacrylat (ADAME) mit einem Quaternisierungsmittel der folgenden Formel (II) in Gegenwart von Wasser:
R - Cl (II),
worin R die oben angegebenen Bedeutungen aufweist, **dadurch gekennzeichnet, dass** die Reaktion in einem röhrenförmigen Reaktor kontinuierlich durchgeführt wird, wobei das Quaternisierungsmittel am Boden des Reaktors und ADAME und Wasser am Kopf des Reaktors zugeführt werden und wobei die Umsetzung bei einer Temperatur von 35 bis 60° C und bei einem Druck von 10 bis 20 bar durchgeführt wird.

2. Verfahren nach Anspruch 1, **dadurch gekennzeichnet, dass** die Umsetzung bei einer Temperatur von 40 bis 50° C durchgeführt wird.

3. Verfahren nach einem der Ansprüche 1 und 2, **dadurch gekennzeichnet, dass** die Umsetzung bei einem Druck von 12 bis 16 bar durchgeführt wird.

4. Verfahren nach einem der Ansprüche 1 bis 3, **dadurch gekennzeichnet, dass** die Umsetzung mit einem Molverhältnis von CH₃Cl und ADAME von 1 bis 1,5 durchgeführt wird.

5. Verfahren nach Anspruch 4, **dadurch gekennzeichnet, dass** die Umsetzung mit einem Molverhältnis von Quaternisierungsmittel und ADAME von 1,02 bis 1,15 durchgeführt wird.

6. Verfahren nach einem der Ansprüche 1 bis 5, **dadurch gekennzeichnet, dass** die Umsetzung mit einem Durchsatzverhältnis Wasser/Quaternisierungsmittel von 0,3 bis 1,2 und insbesondere 0,5 bis 0,9 bei der Zufuhr durchgeführt wird.

7. Verfahren nach einem der Ansprüche 1 bis 6, **dadurch gekennzeichnet, dass** die Umsetzung mit einer Verweilzeit von 2 bis 6 Stunden durchgeführt wird.

8. Verfahren nach Anspruch 7, **dadurch gekennzeichnet, dass** die Umsetzung mit einer Verweilzeit von 2,5 bis 3,5 Stunden durchgeführt wird.

9. Verfahren nach einem der Ansprüche 1 bis 8, **dadurch gekennzeichnet, dass** es in Gegenwart mindestens eines Stabilisierungsmittels durchgeführt wird, das insbesondere unter 3,5-Di*t*-Butyl-4-hydroxytoluol, Hydrochinonmethylether, Hydrochinon, Catechin, *t*-Butylcatechin und den Gemischen dieser Stabilisierungsmittel ausgewählt ist, wobei der Mengenanteil des Stabilisierungsmittels oder der Stabilisierungsmittel insbesondere im Bereich von 20 bis 200 ppm und vorzugsweise 100 bis 1200 ppm, bezogen auf die wässrige Lösung des quartären Salzes (I), liegt.

10. Verfahren nach Anspruch 9, **dadurch gekennzeichnet, dass** es ferner in Gegenwart mindestens eines Maskierungsmittel für Metalle durchgeführt wird, das insbesondere unter Diethylentriaminpentaessigsäure, dem Pentanatriumsalz von Diethylentriaminpentaessigsäure, N-Hydroxyethyl-ethylendiamintriessigsäure und dem Trinatriumsalz von N-Hydroxyethyl-ethylendiamintriessigsäure durchgeführt wird, wobei der Mengenanteil des Maskierungsmittels oder der Maskierungsmittel insbesondere im Bereich von 1 bis 100 ppm und vorzugsweise 5 bis 30 ppm, bezogen auf die wässrige Lösung des quartären Salzes (I), liegt.

## Claims

1. Process for the manufacture of an aqueous solution of the unsaturated quaternary ammonium salt corresponding to the following formula (I): 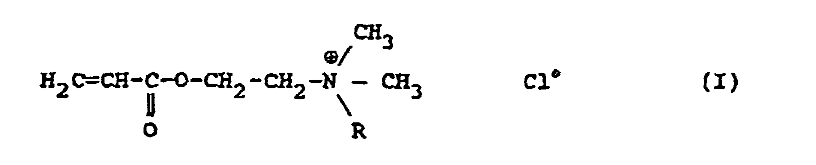 in which R represents a methyl or benzyl radical,
by reaction, in the presence of water, of N,N-dimethylaminoethyl acrylate (DAMEA) with a quaternizing agent of formula (II):
R - Cl (II)
in which R is as defined above,
**characterized in that** the said reaction is carried out continuously in a tubular reactor, with introduction of the quaternizing agent in the reactor bottom and introduction of the DAMEA and the water in the reactor top, the said reaction being carried out at a temperature of 35 to 60°C and under a pressure of 10 to 20 bar.

2. Process according to Claim 1, **characterized in that** the reaction is carried out at a temperature of 40 to 50°C.

3. Process according to either of Claims 1 and 2, **characterized in that** the reaction is carried out under a pressure of 12 to 16 bar.

4. Process according to one of Claims 1 to 3, **characterized in that** the reaction is carried out with a molar ratio of CH₃Cl to the DAMEA which is between 1 and 1.5.

5. Process according to Claim 4, **characterized in that** the reaction is carried out with a molar ratio of the quaternizing agent to the DAMEA which is between 1.02 and 1.15.

6. Process according to one of Claims 1 to 5, **characterized in that** the reaction is carried out with a ratio of the throughput for the introduction of water/throughput for the introduction of quaternizing agent of between 0.3 and 1.2, in particular between 0.5 and 0.9.

7. Process according to one of Claims 1 to 6, **characterized in that** the reaction is carried out with a residence time of 2 to 6 hours.

8. Process according to Claim 7, **characterized in that** the reaction is carried out with a residence time of 2.5 to 3.5 hours.

9. Process according to one of Claims 1 to 8, **characterized in that** it is carried out in the presence of at least one stabilizer chosen in particular from 3,5-di(tert-butyl)-4-hydroxytoluene, hydroquinone methyl ether, hydroquinone, catechol, tert-butylcatechol and the mixtures of these stabilizers, the content of stabilizing agent(s) being in particular from 20 to 2 000 ppm, preferably from 100 to 1 200 ppm, with respect to the aqueous solution of quaternary salt (I).

10. Process according to Claim 9, **characterized in that** it is carried out, in addition, in the presence of at least one sequestering agent for metals chosen in particular from diethylenetriaminepentaacetic acid, the pentasodium salt of diethylenetriaminepentaacetic acid, N-(hydroxyethyl)ethylenediaminetriacetic acid and the trisodium salt of N-(hydroxyethyl)ethylene-diaminetri-acetic acid, the content of sequestering agent(s) being in particular from 1 to 100 ppm, preferably from 5 to 30 ppm, with respect to the aqueous solution of quaternary salt (I).
